# EUROPÄISCHE PATENTSCHRIFT

(11) **EP 3 633 384 B1**
(45) Veröffentlichungstag und Bekanntmachung des Hinweises auf die Patenterteilung: **09.12.2020**
(21) Anmeldenummer: 18198729.8
(22) Anmeldetag: 04.10.2018
(51) Int. Cl.: G01N 35/00, F16G 11/10, H02G 3/30, A61M 5/14

(54) **SCHLAUCHFÜHRUNG FÜR EIN LABORAUTOMATISIERUNGSSYSTEM**
HOSE GUIDE FOR A LABORATORY AUTOMATION SYSTEM
GUIDAGE DE TUYAU POUR UN SYSTÈME D'AUTOMATISATION DE LABORATOIRE

(43) Veröffentlichungstag der Anmeldung: 08.04.2020
(73) Patentinhaber: Tecan Trading AG, 8708 Männedorf (CH)
(72) Erfinder: Kuster, Martin, 8733 Eschenbach (CH)
(74) Vertreter: Qip Patentanwälte Dr. Kuehn & Partner mbB

(56) Entgegenhaltungen:
- US-A1- 2013 138 044
- US-A1- 2014 049 144
- US-A1- 2014 103 171
- US-B1- 6 382 569

## Beschreibung

Die Erfindung betrifft eine Schlauchführung für ein Laborautomatisierungssystem, eine Laborautomatisierungssystem-Komponente sowie ein Laborautomatisierungssystem.

### HINTERGRUND DER ERFINDUNG

Laborautomatisierungssysteme werden zum Automatisieren von Tätigkeiten eines Laborassistenten verwendet. Beispielsweise können mit einem Laborautomatisierungssystem Proben pipettiert, mit Chemikalien und/oder Reagenzien gemischt und die dabei auftretenden Reaktionen analysiert werden. Das Laborautomatisierungssystem kann dazu eine Pipettiervorrichtung aufweisen, die über Schläuche mit einer Pumpe verbunden ist. Die Pipettiervorrichtung kann beispielsweise in Reagenzgläser oder eine Pipettierplatte abgesenkt werden und die Pumpe kann mithilfe der Schläuche Flüssigkeiten aus diesen Behältern aspirieren und dispensieren. Ist die Pipettiervorrichtung beweglich gelagert, müssen die Schläuche in der Regel längere Strecken entlang von Armen und/oder Gehäuseteilen geführt werden. Daher werden die Schläuche beispielsweise mit Klammern an verschiedenen Komponenten des Laborautomatisierungssystems fixiert. Dabei ist es wichtig, dass die Schläuche bei Bewegung der Pipettiervorrichtung nicht geknickt oder beschädigt werden können.

Schlauchführungen für medizinische Behandlungsvorrichtungen sind aus US 2013/138044 A1 und US 6, 382, 569 B1 bekannt. Kabelführungen mit Führungsöffnungen sind aus US 2014/049144 A1 und US 2014/103171 A1 bekannt.

### ZUSAMMENFASSUNG DER ERFINDUNG

Es ist Aufgabe der Erfindung, Schläuche in einem Laborautomatisierungssystem sicher und kostengünstig zu verlegen. Weiter kann mit der Erfindung eine Zugsentlastung der Schläuche gewährleistet werden.

Diese Aufgabe wird durch den Gegenstand der unabhängigen Ansprüche gelöst. Weitere Ausführungsformen der Erfindung ergeben sich aus den abhängigen Ansprüchen und aus der folgenden Beschreibung.

Ein Aspekt der Erfindung betrifft eine Schlauchführung für ein Laborautomatisierungssystem. Ein Laborautomatisierungssystem kann dabei ein Gerät sein, das dazu ausgeführt ist, Pipettiervorgänge zu automatisieren.

Dazu kann das Laborautomatisierungssystem mit einem Werktisch eine ebene Fläche bereitstellen, auf der Behälter und/oder Analysegeräte abgestellt werden können. Weiter kann das Laborautomatisierungssystem einen Pipettierarm aufweisen, der bezüglich des Werktischs beweglich gelagert sein kann. An dem Pipettierarm kann eine Pipettiervorrichtung befestigt sein, die mit dem Pipettierarm bewegt werden kann. Die Pipettiervorrichtung, die eine Mehrzahl von Pipetten tragen kann, kann über mehrere Schläuche mit einer Pumpe verbunden sein, die beispielsweise an dem Werktisch befestigt ist.

Die Schlauchführung kann ein Bestandteil des Laborautomatisierungssystems sein und/oder von einer Komponente des Laborautomatisierungssystems, wie etwa einem Pumpengehäuse, einem Pipettierarm, usw., bereitgestellt werden. Die Schlauchführung kann dazu ausgeführt sein, die Schläuche zu führen, an einer Stelle des Laborautomatisierungssystems zu fixieren und/oder umzulenken.

Gemäß einer Ausführungsform der Erfindung umfasst die Schlauchführung eine Platte mit einer Führungsöffnung, wobei die Führungsöffnung zwei Teilöffnungen aufweist, die durch einen Steg getrennt sind und die durch einen Schlitz verbunden sind. Die Teilöffnungen weisen an voneinander weg weisenden Rändern gegenüberliegende Aussparungen zum Führen jeweils eines Schlauches auf.

Die Platte kann eine Kunststoffplatte oder eine Metallplatte sein. Die Platte kann ein Blech, wie etwa ein Kunststoffblech oder ein Metallblech sein. Die Platte kann eine Kunststoffplatte mit Faserverstärkung, beispielsweise mit Kohlefasern, sein. Die Platte kann eine Verschalung einer Komponente des Laborautomatisierungssystems sein. In der Umgebung der Schlauchführung kann die Platte eben sein. Es ist möglich, dass die Schlauchführung in einem ebenen Bereich einer abgewinkelten Platte angeordnet ist.

Die Führungsöffnung umfasst zwei Teilöffnungen, die über einen Schlitz miteinander verbunden sind. Die Führungsöffnung kann auf einfache Art und Weise durch Stanzen und/oder Schneiden der Platte erzeugt werden. Es ist nicht notwendig, weitere Elemente für eine Schlauchführung an den gewünschten Stellen des Laborautomatisierungssystems anzubringen. Damit können Material und Arbeitsschritte eingespart werden.

Ein Schlauch kann durch den Schlitz in die Führungsöffnung und insbesondere zwei Aussparungen eingelegt werden und hinter den Steg geschoben werden. Der Steg kann verhindern, dass der Schlauch aus der Führungsöffnung fällt. Der Steg kann quer zu einer Führungsrichtung verlaufen, in die die Schläuche durch die Führungsöffnung geführt werden. Die Führungsrichtung kann durch zwei gegenüberliegende Aussparungen, die für einen Schlauch bestimmt sind, definiert sein.

Der Schlitz kann eine Breite aufweisen, die etwas (beispielsweise bis zu 10%) größer ist als der Durchmesser eines Schlauchs. Die Aussparungen und/oder deren Kanten können ein seitliches Verrutschen des Schlauchs und/oder ein Verrutschen des Schlauchs entlang seiner Längsrichtung verhindern. Die Aussparungen können eine Breite aufweisen, die dem Durchmesser eines Schlauchs entspricht.

Die Aussparungen einer Teilöffnung können nebeneinander angeordnet sein und/oder in die gleiche Richtung geöffnet sein. Die Aussparungen der anderen Teilöffnung können in die entgegengesetzte Richtung geöffnet sein.

Gemäß einer Ausführungsform der Erfindung verläuft der Schlitz durch eine Mitte des Stegs. Der Steg kann zweigeteilt sein. Beispielsweise können zwei zungenförmige Abschnitte des Stegs quer zur Führungsrichtung in die Führungsöffnung hineinragen.

Gemäß einer Ausführungsform der Erfindung verläuft der Schlitz an einem Ende des Stegs. Der Steg kann einteilig sein. Beispielsweise kann ein zungenförmiger Abschnitt des Stegs quer zur Führungsrichtung in die Führungsöffnung hineinragen.

Gemäß einer Ausführungsform der Erfindung verläuft der Schlitz schräg zu der Führungsrichtung, die durch zwei gegenüberliegende Aussparungen für einen Schlauch definiert ist. Dies kann erschweren, dass ein Schlauch nach dem Verlegen aus der Führungsöffnung herausfällt.

Gemäß einer Ausführungsform der Erfindung ist der Schlitz U-förmig gekrümmt. Auch dies kann erschweren, dass ein Schlauch nach dem Verlegen aus der Führungsöffnung herausfällt.

Gemäß einer Ausführungsform der Erfindung verbreitert sich der Steg auf den Schlitz zu. Beispielsweise kann eine quer zur Führungsrichtung verlaufende Zunge des Stegs an ihrem Ende L-förmig oder T-förmig sein. Dies ist eine weitere Möglichkeit, zu erschweren, dass ein Schlauch nach dem Verlegen aus der Führungsöffnung herausfällt.

Gemäß einer Ausführungsform der Erfindung sind die Aussparungen in einer Querrichtung bzw. quer zur Führungsrichtung beabstandet zu einem Schlitzbereich des Steges angeordnet. Der Schlitzbereich kann ein Abschnitt des Stegs sein, in dem der Schlitz vorhanden ist. Damit verlaufen verlegte Schläuche neben dem Schlitz hinter dem Steg.

Gemäß einer Ausführungsform der Erfindung verbreitern sich die Aussparungen auf den Steg zu. Beispielsweise können die Aussparungen dreieckig, oval, parabelförmig, ellipsenförmig und/oder polygonal sein. Im Allgemeinen können die Aussparungen an der Seite, die auf den Steg zu weist, breiter als ein Durchmesser eines Schlauchs sein und sich mit vergrößerndem Abstand zum Steg verjüngen, so dass ihre Breite den Durchmesser des Schlauchs unterschreitet.

Gemäß einer Ausführungsform der Erfindung ist die Führungsöffnung in einem Öffnungsbereich der Platte angeordnet, der gegenüber einem umgebenden Bereich erhaben ist. Die Führungsöffnung und der Öffnungsbereich können in einem Arbeitsschritt durch Stanzen, Laser- und/oder Wasserstrahlschneiden und/oder anderen Methoden geformt werden. Hinter dem Öffnungsbereich kann ein zusätzlicher Raum erzeugt werden, durch den die hinter den Steg geklemmten Schläuche verlaufen können, ohne dass Bauelemente hinter der Platte, die eine Verschalung sein kann, beeinträchtigt werden.

Gemäß einer Ausführungsform der Erfindung sind die Teilöffnungen und der Steg in einer Ebene angeordnet, insbesondere in einer anderen Ebene als die Ebene, die durch den Bereich definiert wird, der den Öffnungsbereich umgibt. Damit kann die Klemmwirkung des Stegs auf den Schlauch und/oder mehrere Schläuche sichergestellt werden.

Gemäß einer Ausführungsform der Erfindung weist die Platte eine Mehrzahl von Führungsöffnungen auf. Somit können Schläuche entlang einer größeren Strecke an der Platte fixiert und geführt werden.

Gemäß einer Ausführungsform der Erfindung weist die Platte eine Umlenköffnung auf, die zwei schräg zueinander angeordnete Teilöffnungen aufweist, die durch einen Steg getrennt sind und die durch einen Schlitz verbunden sind. Die Umlenköffnung muss keine Aussparungen aufweisen, die die Schläuche in einer Bewegungsrichtung fixieren. Trotzdem können die Schläuche durch den Steg an der Platte gehalten werden.

Es ist zu verstehen, dass Bestandteile der Umlenköffnung, wie die der Führungsöffnung, zueinander angeordnet und/oder geformt sein können. Beispielsweise kann die Führungsöffnung Aussparungen aufweisen. Es ist auch möglich, dass der Schlitz in der Mitte oder an einem Ende des Stegs angeordnet ist usw.

Gemäß einer Ausführungsform der Erfindung ist die Umlenköffnung in einem Öffnungsbereich der Platte angeordnet, der gegenüber einem umgebenden Bereich erhaben ist, wobei die Teilöffnungen der Umlenköffnung in einem Übergangsbereich zwischen dem Öffnungsbereich und dem umgebenden Bereich angeordnet sind, so dass Ränder der Teilöffnungen in unterschiedlichen Ebenen angeordnet sind. Auf diese Weise klemmt der Steg die Schläuche nicht mehr und die Schläuche können durch die Umlenköffnung gleiten.

Gemäß einer Ausführungsform der Erfindung verläuft der Schlitz der Umlenköffnung durch den Übergangsbereich und den Öffnungsbereich. Dies kann ein Herausfallen der Schläuche aus der Umlenköffnung verhindern.

Ein weiterer Aspekt der Erfindung betrifft eine Komponente für ein Laborautomatisierungssystem. Eine Komponente kann dabei ein Bestandteil des Laborautomatisierungssystems sein, das ein Gehäuse mit einer Platte aufweist. Beispielsweise kann die Komponente eine Pumpe, ein Pipettierarm oder eine Armaufhängung des Pipettierarms sein.

Gemäß einer Ausführungsform der Erfindung umfasst die Komponente eine Schlauchführung und eine Mehrzahl von Schläuchen, die durch die Führungsöffnung der Schlauchführung geführt sind. Die Platte mit der Führungsöffnung kann eine Verschalung der Komponente sein. Jeder der Schläuche kann durch die beiden Teilöffnungen und hinter dem Steg verlaufen und in jeweils zwei gegenüberliegende Aussparungen eingelegt sein.

Ein weiterer Aspekt der Erfindung betrifft ein Laborautomatisierungssystem.

Gemäß einer Ausführungsform der Erfindung umfasst das Laborautomatisierungssystem einen Werktisch, eine am Werktisch befestigte Schiene einen über eine Armaufhängung beweglich an der Schiene befestigten Arm, an dem eine Pipettiervorrichtung über dem Werktisch befestigt ist, und eine Pumpe.

Gemäß einer Ausführungsform der Erfindung umfasst das Laborautomatisierungssystem eine Mehrzahl von Schläuchen, die von der Pumpe zu der Pipettiervorrichtung verlaufen und ein Platte mit einer Schlauchführung, so wie sie oben stehend und unten stehend beschrieben ist. Beispielsweise können die Pumpe, die Armaufhängung und/oder der Pipettierarm die Platte aufweisen.

### KURZE BESCHREIBUNG DER FIGUREN

Im Folgenden werden Ausführungsbeispiele der Erfindung mit Bezug auf die beiliegenden Figuren detailliert beschrieben.
Fig. 1 zeigt schematisch eine perspektivische Ansicht eines Laborautomatisierungssystems gemäß einer Ausführungsform der Erfindung.
Fig. 2 zeigt eine Draufsicht auf eine Schlauchführung gemäß einer Ausführungsform der Erfindung.
Fig. 3 zeigt Umrisse von Schlauchführungen gemäß Ausführungsformen der Erfindung.
Fig. 4 zeigt eine perspektivische Ansicht einer Pumpe mit einer Schlauchführung gemäß einer Ausführungsform der Erfindung.
Fig. 5 zeigt eine Draufsicht auf eine Schlauchführung gemäß einer Ausführungsform der Erfindung.
Fig. 6 zeigt eine perspektivische Ansicht einer Armaufhängung mit einer Schlauchführung gemäß einer Ausführungsform der Erfindung.
Fig. 7 zeigt eine Draufsicht auf eine Schlauchführung gemäß einer Ausführungsform der Erfindung.
Fig. 8 zeigt eine perspektivische Ansicht eines Pipettierarms mit einer Schlauchführung gemäß einer Ausführungsform der Erfindung.

Die in den Figuren verwendeten Bezugszeichen und ihre Bedeutung sind in zusammenfassender Form in der Liste der Bezugszeichen aufgeführt. Grundsätzlich sind identische oder ähnliche Teile mit den gleichen Bezugszeichen versehen.

### DETAILLIERTE BESCHREIBUNG VON AUSFÜHRUNGSBEISPIELEN

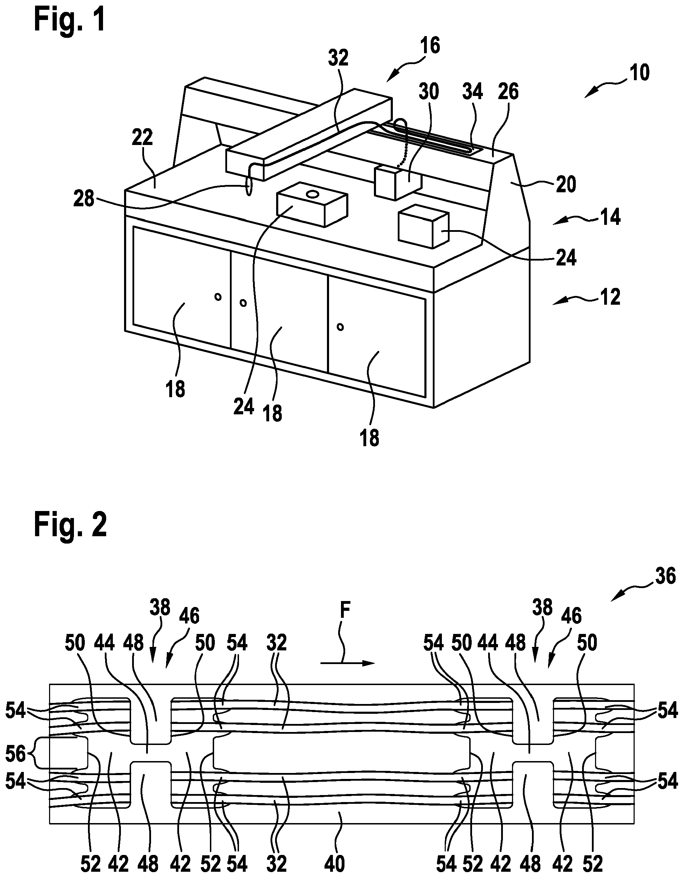

Fig. 1 zeigt ein Laborautomatisierungssystem 10, das einen Unterbau 12, einen auf dem Unterbau 12 abgestellten Werktisch 14 und einen an dem Werktisch 14 befestigten Pipettierarm 16 umfasst. Der Unterbau 12 kann einen oder mehrere Schränke 18 umfassen und/oder kann den Werktisch 14 auf einer Höhe halten, so dass auf den Werktisch 14 angenehm von einer Bedienperson zugegriffen werden kann. Der Unterbau 12 ist optional. Das Laborautomatisierungssystem 10 kann beispielsweise auch direkt auf einen konventionellen Labortisch gestellt werden.

Der Werktisch 14 weist einen Rahmen 20 auf, auf dem eine Arbeitsplatte 22 befestigt ist, auf der wiederum Behälter 24, wie etwa Behälter für Wegwerf-Pipettierspitzen, Behälter für Proben, Behälter für Chemikalien, Reagenzien, und/oder Pipettierplatten usw., fixiert bzw. verankert sein können.

Der Werktisch 14 weist weiter eine Schiene 26 auf, die über der Arbeitsplatte 22 angeordnet ist und die parallel zu der Arbeitsplatte verläuft. An dieser Schiene 26 ist der Pipettierarm 16 beweglich befestigt. Mithilfe eines oder mehrerer Motoren kann der Pipettierarm 16 entlang der Schiene 26 bewegt werden und/oder kann eine vom Pipettierarm 16 getragene Pipettiervorrichtung 28 in drei Dimensionen über der Arbeitsplatte 22 bewegt werden.

Mit einer in das Laborautomatisierungssystem 10 integrierten Pumpe 30, die beispielsweise am Werktisch 14 befestigt ist, können Flüssigkeiten aus Behältern 24 mit der Pipettiervorrichtung 28 aspiriert und dispensiert werden. Die Pipettiervorrichtung 28 kann dazu mehrere Pipettierspitzen aufweisen, die über Schläuche 32 mit der Pumpe 30 verbunden sind.

Die Schläuche 32 werden von der Pumpe aus über die Schiene 26, den Pipettierarm 16 entlang zu der Pipettiervorrichtung 28 geführt. In der Schiene 26 können die Schläuche 32 durch eine Schleppkette 34 geführt sein, um so die Bewegungen des Pipettierarms 16 auszugleichen.

In den folgenden Figuren wird beschrieben, wie die Schläuche 32 an verschiedenen Gehäuseteilen des Laborautomatisierungssystems 10 fixiert und/oder befestigt sein können.

Die Fig. 2 zeigt eine Schlauchführung 36 mit zwei Führungsöffnungen 38, die in einer Platte 40, beispielsweise einem Blech, gebildet sind. Die Platte 40 kann eine Verschalung einer Komponente des Laborautomatisierungssystems 10 aus der Fig. 1 sein, wie etwa der Pumpe 30 oder dem Pipettierarm 16.

Jede der Führungsöffnungen 38 weist zwei Teilöffnungen 42 mit einer rechteckigen Grundform auf, die über einen Schlitz 44 miteinander verbunden sind. Der Schlitz 44 verläuft in einer Führungsrichtung F, in der jeder der Schläuche 32 durch die Führungsöffnungen 38 geführt wird. Zwischen den Teilöffnungen 42 verläuft ein Steg 46, durch den der Schlitz 44 in Führungsrichtung F verläuft. Auf diese Weise umfasst der Steg 46 zwei Zungen 48, die quer zu der Führungsrichtung F aufeinander zu verlaufen. Durch die Zungen 48 werden die Schläuche 32 in Führungsöffnungen 38 geklemmt.

Der Steg 46 stellt für jede der Teilöffnungen 42 einen innen liegenden Rand 50 bereit. Gegenüberliegend zu dem Rand 50 befindet sich ein außen liegender Rand 52, an dem eine Mehrzahl von Aussparungen 54 gebildet ist. Die Ränder 50 und/oder die Ränder 52 verlaufen im Wesentlichen parallel.

In die Aussparungen 54, die in einer Reihe entlang des Rands 52 angeordnet sind, ist jeweils ein Schlauch 32 eingelegt. Die Aussparungen 54 verhindern eine Bewegung des Schlauchs 32 quer zu der Führungsrichtung F. Die Aussparungen 54 weisen eine Öffnung auf, die auf den Steg 46 zu weist und verjüngen sich mit sich vergrößerndem Abstand zum Steg 46.

Die Aussparungen 54 sind in einer Querrichtung orthogonal zu der Führungsrichtung F beabstandet zu einem Schlitzbereich 56 angeordnet, so dass der jeweilige Schlauch 32 hinter dem Steg 46 neben dem Schlitz 44 durch die Führungsöffnung 38 verläuft.

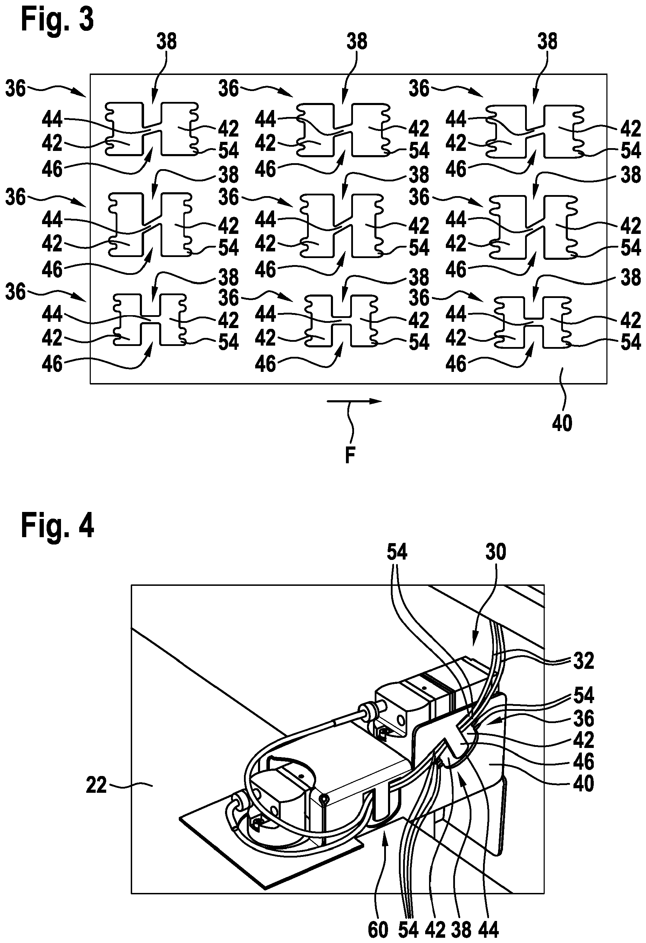

Die Fig. 3 zeigt weitere Ausführungsformen von Schlauchführungen 36 bzw. Führungsöffnungen 38. Es ist gezeigt, dass die Aussparungen 54 oval (links), dreieckig (mittig) und parabelförmig (rechts) sein können. Weiter kann der Schlitz 44 schräg (oben) oder parallel zu der Führungsrichtung F orientiert sein.

Die Klemmwirkung einer Führungsöffnungen 38 kann abhängig von der Plattendicke der Platte 40, von dem Abstand der Aussparungen 54 in Richtung F, von der Stegbreite des Stegs 46 in Richtung F und/oder dem Verhältnis der Stegbreite zu dem Abstand der Aussparungen 54 sein. In der Fig. 3 sind Führungsöffnungen 38 gezeigt, die sich in dem Abstand der Aussparungen 54 und der Stegbreite unterscheiden.

Die Fig. 4 zeigt eine Pumpe 30 genauer, wie sie beispielsweise in dem Laborautomatisierungssystem 10 aus der Fig. 1 verbaut sein kann. Die Pumpe 30 weist ein Gehäuse 58 auf, das die Platte 40 mit einer Schlauchführung 36 umfasst. Die Schläuche 32 sind mit der Pumpe 30 verbunden und sind anschließend durch eine Führungsöffnung 60 ohne Aussparungen und eine Führungsöffnung 38 mit Aussparungen 54 geführt. Von dort verlaufen sie weiter nach oben in Richtung Schiene 26.

Die Führungsöffnung 38 weist einen U-förmigen Schlitz 44 auf, der mit einem Seitenrand der Teilöffnungen 42 verbunden ist.

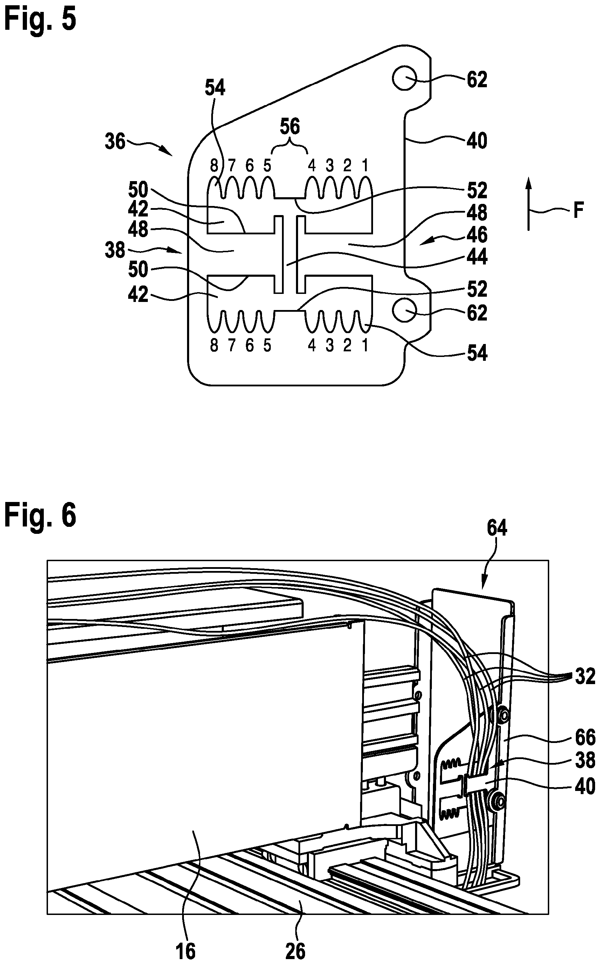

Die Fig. 5 zeigt eine Schlauchführung 36, die in einer gesonderten Platte 40 realisiert ist, das über Öffnungen 62 an einem weiteren Gehäuseteil befestigt werden kann. Bei der Fig. 5 weist der Steg 46 Zungen 48 auf, die ein sich auf den Schlitz 44 zu verbreiterndes Ende aufweisen. Weiter ist gezeigt, dass die Aussparungen 54 mittels Öffnungen in der Platte 40 in der Form von Ziffern/Buchstaben gekennzeichnet werden können.

Die Fig. 3 zeigt eine Armaufhängung 64 genauer, wie sie beispielsweise in dem Laborautomatisierungssystem 10 aus der Fig. 1 verbaut sein kann. Über die Armaufhängung 64 kann der Pipettierarm 16 beweglich mit der Schiene 26 verbunden sein. Die Armaufhängung 64 umfasst ein Gehäuse 66, in das die in der Fig. 5 gezeigte Platte 40 verbaut ist.

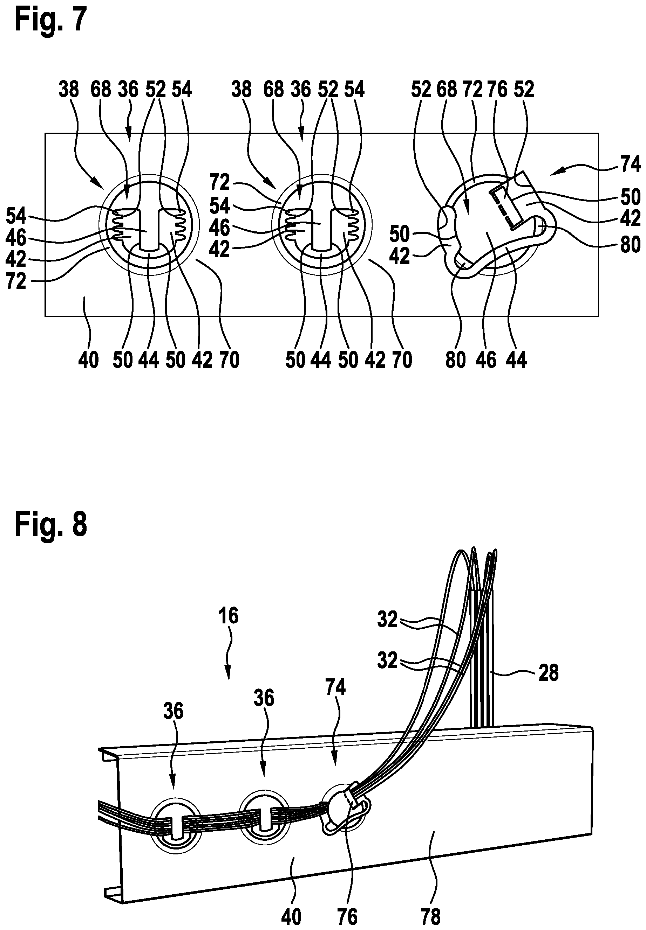

Die Fig. 7 zeigt eine weitere Ausführungsform einer Schlauchführung 36 bzw. Führungsöffnung 38, die in der in der Fig. 7 gezeigten Platte 40 zweimal realisiert ist. Hier ist der Schlitz 44 U-förmig gekrümmt und mit einem Seitenrand der Teilöffnungen 42 verbunden. Die Führungsöffnung ist in einem kreisförmigen Öffnungsbereich 68 angeordnet, der gegenüber einem umgebenden Bereich 70 erhaben ist. Der Bereich 68 und der Bereich 70 sind jeweils eben und sind über einen gegenüber diesen Bereichen schräg verlaufenden Übergangsbereich 72 verbunden.

Die Führungsöffnung 38 und der Steg 46 sind in dem Öffnungsbereich 68 realisiert und befinden sich damit in einer anderen Ebene als eine durch den Bereich 70 definierten Ebene.

Die Fig. 7 zeigt weiter eine Umlenköffnung 74, die in der Platte 40 gebildet ist. Die Umlenköffnung 74 weist zwei schräg zueinander angeordnete Teilöffnungen 42 auf, die durch einen Steg 46 getrennt sind und die durch einen Schlitz 44 verbunden sind. Die Teilöffnungen 42 werden seitlich durch zwei Zungen 80 begrenzt, die von dem Steg 46 bereitgestellt sind. Auf diese Weise können Schläuche nicht aus den Teilöffnungen 42 herausgleiten. Die Umlenköffnung 74 ist in einem Öffnungsbereich 68 der Platte 40 angeordnet, der gegenüber dem umgebenden Bereich 70 erhaben ist. Der Steg 46 reicht dabei bis in den Übergangsbereich 72 und den Umgebungsbereich 70. Die Zungen 80 können sich von dem Bereich 68 bis in den Bereich 70 erstrecken. Der Schlitz 44 der Umlenköffnung 74 verläuft durch den Umgebungsbereich 70, den Übergangsbereich 72 und den Öffnungsbereich 68. Die dreidimensionale Form, die durch die Bereiche 70, 72, 74 gebildet ist, kann wie bei den Führungsöffnungen 38 mit einem Napfwerkzeug erzeugt werden.

Die Teilöffnungen 42 der Umlenköffnung 74 sind in dem Übergangsbereich 72 zwischen den Bereichen 68, 70 angeordnet, so dass ihre Ränder 50, 52 in unterschiedlichen Ebenen angeordnet sind.

An der Teilöffnung 42, bei der die Schläuche 32 aus der Umlenköffnung 74 austreten, ist bei dem Innenrand 50 ein Flügel 76 vorhanden, der gegenüber der Ebene des Öffnungsbereichs 68 nach außen abgewinkelt ist. Dies kann die Reibung zwischen den Schläuchen 32 und der Umlenköffnung 74 vermindern.

Die Fig. 8 zeigt einen Pipettierarm 16 genauer, wie er beispielsweise in dem Laborautomatisierungssystem 10 aus der Fig. 1 verbaut sein kann. Der Pipettierarm 16 umfasst ein Gehäuse 78 mit einer Platte 40, in dem zwei Schlauchführungen 36 und eine Umlenköffnung 74 ausgeführt sind, so wie sie in der Fig. 7 gezeigt sind. Die Schläuche 32 verlaufen durch die beiden Schlauchführungen 36, wo sie an dem Gehäuse 78 fixiert sind, und anschließend durch die Umlenköffnung 74, in der sie sich frei bewegen können. Von dort aus verlaufen sie zu der Pipettiervorrichtung 28, die sich nach oben und unten bewegen kann.

Ergänzend ist darauf hinzuweisen, dass "umfassend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

### LISTE DER BEZUGSZEICHEN

- 10: Laborautomatisierungssystem
- 12: Unterbau
- 14: Werktisch
- 16: Pipettierarm
- 18: Schrank
- 20: Rahmen
- 22: Arbeitsplatte
- 24: Behälter
- 26: Schiene
- 28: Pipettiervorrichtung
- 30: Pumpe
- 32: Schlauch
- 34: Schleppkette
- 36: Schlauchführung
- 38: Führungsöffnung
- 40: Platte
- 42: Teilöffnung
- 44: Schlitz
- F: Führungsrichtung
- 46: Steg
- 48: Zunge
- 50: Rand
- 52: Rand
- 54: Aussparung
- 56: Schlitzbereich
- 58: Gehäuse
- 60: Führungsöffnung
- 62: Öffnung
- 64: Armaufhängung
- 66: Gehäuse
- 68: Öffnungsbereich
- 70: Umgebungsbereich
- 72: Übergangsbereich
- 74: Umlenköffnung
- 76: Flügel
- 78: Gehäuse
- 80: Zunge

## Patentansprüche

1. Schlauchführung (36) für ein Laborautomatisierungssystem (10),
wobei die Schlauchführung (36) eine Platte (40) mit einer Führungsöffnung (38) umfasst;
wobei die Führungsöffnung (38) zwei Teilöffnungen (42) aufweist, die durch einen Steg (46) getrennt sind und die durch einen Schlitz (44) verbunden sind; **dadurch gekennzeichnet, dass** die Teilöffnungen (42) an voneinander weg weisenden Rändern (52) gegenüberliegende Aussparungen (54) zum Führen jeweils eines Schlauches (32) aufweisen.

2. Schlauchführung (36) nach Anspruch 1,
wobei der Schlitz (44) durch eine Mitte des Stegs (46) verläuft; oder
wobei der Schlitz (44) an einem Ende des Stegs (46) verläuft.

3. Schlauchführung (36) nach Anspruch 1 oder 2,
wobei der Schlitz (44) schräg zu einer Führungsrichtung (F) verläuft, die durch zwei gegenüberliegende Aussparungen (54) für einen Schlauch (32) definiert ist.

4. Schlauchführung (36) nach einem der vorhergehenden Ansprüche,
wobei der Schlitz (44) U-förmig gekrümmt ist.

5. Schlauchführung (36) nach einem der vorhergehenden Ansprüche,
wobei der Steg (46) sich auf den Schlitz (44) zu verbreitert.

6. Schlauchführung (36) nach einem der vorhergehenden Ansprüche,
wobei die Aussparungen (54) in einer Querrichtung beabstandet zu einem Schlitzbereich (56) des Steges (46) angeordnet sind, in dem der Schlitz (44) vorhanden ist.

7. Schlauchführung (36) nach einem der vorhergehenden Ansprüche,
wobei die Aussparungen (54) sich auf den Steg (46) zu verbreitern.

8. Schlauchführung (36) nach einem der vorhergehenden Ansprüche,
wobei die Aussparungen (54) dreieckig, oval, parabelförmig, ellipsenförmig und/oder polygonal sind.

9. Schlauchführung (36) nach einem der vorhergehenden Ansprüche,
wobei die Führungsöffnung (38) in einem Öffnungsbereich (68) der Platte (40) angeordnet ist, der gegenüber einem umgebenden Bereich (70) erhaben ist; und/oder
wobei die Teilöffnungen (42) und der Steg (46) in einer Ebene angeordnet sind.

10. Schlauchführung (36) nach einem der vorhergehenden Ansprüche,
wobei die Platte (40) eine Mehrzahl von Führungsöffnungen (38) aufweist.

11. Schlauchführung (36) nach einem der vorhergehenden Ansprüche,
wobei die Platte (40) eine Umlenköffnung (74) aufweist, die zwei schräg zueinander angeordnete Teilöffnungen (42) aufweist, die durch einen Steg (46) getrennt sind und die durch einen Schlitz (44) verbunden sind.

12. Schlauchführung (36) nach Anspruch 11,
wobei die Umlenköffnung (74) in einem Öffnungsbereich (68) der Platte (40) angeordnet ist, der gegenüber einem umgebenden Bereich (70) erhaben ist; und/oder
wobei die Teilöffnungen (42) der Umlenköffnung (74) in einem Übergangsbereich (72) zwischen dem Öffnungsbereich (68) und dem umgebenden Bereich (70) angeordnet sind, so dass Ränder (50, 52) der Teilöffnungen (42) in unterschiedlichen Ebenen angeordnet sind.

13. Schlauchführung (36) nach Anspruch 11 oder 12,
wobei der Schlitz (44) der Umlenköffnung (74) durch den Übergangsbereich (72) und den Öffnungsbereich (68) verläuft.

14. Laborautomatisierungssystem-Komponente (16, 30, 64), umfassend:
eine Schlauchführung (36) nach einem der vorhergehenden Ansprüche,
eine Mehrzahl von durch die Führungsöffnung (38) geführten Schläuchen (32);
wobei die Platte (40) mit der Führungsöffnung (38) eine Verschalung der Komponente (16, 30, 64) ist.

15. Laborautomatisierungssystem (10), umfassend:
einen Werktisch (14);
eine am Werktisch befestigte Schiene (26);
einen über eine Armaufhängung (64) beweglich an der Schiene (26) befestigten Pipettierarm (16), an dem eine Pipettiervorrichtung (28) über dem Werktisch (14) befestigt ist;
eine Pumpe (30);
eine Mehrzahl von Schläuchen (32), die von der Pumpe (30) zu der Pipettiervorrichtung (28) verlaufen;
eine Platte (40) mit einer Schlauchführung (36) nach einem der Ansprüche 1 bis 13 für die Schläuche.

## Claims

1. Tube guide (36) for a laboratory automation system (10), wherein the tube guide (36) comprises a plate (40) having a guide opening (38);
wherein the guide opening (38) comprises two part openings (42) that are separated by a crosspiece (46) and that are connected by means of a slot (44);
**characterised in that** the part openings (42) comprise recesses (54) that lie opposite one another on edges (52) that face away from one another in each case for guiding a tube (32).

2. Tube guide (36) according to Claim 1,
wherein the slot (44) extends through a centre of the crosspiece (46); or
wherein the slot (44) extends on one end of the crosspiece (46).

3. Tube guide (36) according to Claim 1 or 2,
wherein the slot (44) extends at an incline with respect to a guiding direction (F) that is defined by two recesses (54) that lie opposite one another for a tube (32) .

4. Tube guide (36) according to one of the preceding claims,
wherein the slot (44) is curved in a U-shape.

5. Tube guide (36) according to one of the preceding claims,
wherein the crosspiece (46) widens towards the slot (44).

6. Tube guide (36) according to one of the preceding claims,
wherein the recesses (54) are arranged spaced in a transverse direction with respect to a slot region (56) of the cross piece (46) in which the slot (44) is provided.

7. Tube guide (36) according to one of the preceding claims,
wherein the recesses (54) widen towards the crosspiece (46) .

8. Tube guide (36) according to one of the preceding claims,
wherein the recesses (54) are triangular, oval, parabolic, elliptical and/or polygonal.

9. Tube guide (36) according to one of the preceding claims,
wherein the guide opening (38) is arranged in an opening region (68) of the plate (40) that is raised with respect to the surrounding region (70);
and/or
wherein the part openings (42) and the crosspiece (46) are arranged in a plane.

10. Tube guide (36) according to one of the preceding claims,
wherein the plate (40) comprises a plurality of guide openings (38).

11. Tube guide (36) according to one of the preceding claims,
wherein the plate (40) comprises a deflecting opening (74) that comprises two part openings (42) that are arranged at an incline with respect to one another and said part openings are separated by means of a crosspiece (46) and are connected by means of a slot (44).

12. Tube guide (36) according to Claim 11,
wherein the deflecting opening (74) is arranged in an opening region (68) of the plate (40) that is raised with respect to a surrounding region (70);
and/or
wherein the part openings (42) of the deflecting opening (74) are arranged in a transition region (72) between the opening region (68) and the surrounding region (70) with the result that edges (50, 52) of the part openings (42) are arranged in different planes.

13. Tube guide (36) according to Claim 11 or 12,
wherein the slot (44) of the deflecting opening (74) extends through the transition region (72) and the opening region (68).

14. Laboratory automation system component (16, 30, 64), comprising:
a tube guide (36) according to one of the preceding claims,
a plurality of tubes (32) that are guided through the guide opening (38);
wherein the plate (40) having the guide opening (38) is a casing of the component (16, 30, 64).

15. Laboratory automation system (10) comprising:
a work table (14);
a rail (26) that is fastened to the work table;
a pipetting arm (16) that is movably fastened by way of an arm mounting bracket (64) to the rail (26) and a pipetting device (28) is fastened above the work table (14) to said pipetting arm;
a pump (30);
a plurality of tubes (32) that extend from the pump (30) to the pipetting device (28);
a plate (40) having a tube guide (36) according to one of claims 1 to 13 for the tubes.

## Revendications

1. Guidage de tuyau (36) pour un système d'automatisation de laboratoire (10),
le guidage de tuyau (36) comprenant une plaque (40) dotée d'une ouverture de guidage (38) ;
l'ouverture de guidage (38) comportant deux ouvertures partielles (42), qui sont séparées par une partie formant pont (46) et qui sont reliées par une fente (44) ; **caractérisé en ce que**
les ouvertures partielles (42) comportent, sur des bords (52) orientés dans des directions opposées l'un par rapport à l'autre, des évidements (54) opposés pour guider respectivement un tuyau (32).

2. Guidage de tuyau (36) selon la revendication 1,
dans lequel la fente (44) s'étend à travers le milieu de la partie formant pont (46) ; ou
dans lequel la fente (44) s'étend à une extrémité de la partie formant pont (46).

3. Guidage de tuyau (36) selon la revendication 1 ou 2,
dans lequel la fente (44) s'étend de manière oblique par rapport à une direction de guidage (F), qui est définie pour un tuyau (32) par deux évidements (54) opposés.

4. Guidage de tuyau (36) selon l'une des revendications précédentes,
dans lequel la fente (44) est courbée en forme de U.

5. Guidage de tuyau (36) selon l'une des revendications précédentes,
dans lequel la partie formant pont (46) s'élargit en direction de la fente (44).

6. Guidage de tuyau (36) selon l'une des revendications précédentes,
dans lequel les évidements (54) sont disposés espacés dans une direction transversale par rapport à une zone de fente (56) de la partie formant pont (46), dans laquelle se trouve la fente (44).

7. Guidage de tuyau (36) selon l'une des revendications précédentes,
dans lequel les évidements (54) s'élargissent en direction de la partie formant pont (46).

8. Guidage de tuyau (36) selon l'une des revendications précédentes,
dans lequel les évidements (54) sont triangulaires, ovales, en forme de parabole, en forme d'ellipse et/ou polygonaux.

9. Guidage de tuyau (36) selon l'une des revendications précédentes,
dans lequel l'ouverture de guidage (38) est disposée dans une zone d'ouverture (68) de la plaque (40), qui est en relief par rapport à une zone (70) environnante ; et/ou dans lequel les ouvertures partielles (42) et la partie formant pont (46) sont disposées dans un même plan.

10. Guidage de tuyau (36) selon l'une des revendications précédentes,
dans lequel la plaque (40) comporte une pluralité d'ouvertures de guidage (38).

11. Guidage de tuyau (36) selon l'une des revendications précédentes,
dans lequel la plaque (40) comporte une ouverture de changement de direction (74), qui comporte deux ouvertures partielles (42) disposées de manière oblique l'une par rapport à l'autre, qui sont séparées par une partie formant pont (46) et qui sont reliées par une fente (44).

12. Guidage de tuyau (36) selon la revendication 11,
dans lequel l'ouverture de changement de direction (74) est disposée dans une zone d'ouverture (68) de la plaque (40), qui est en relief par rapport à une zone (70) environnante ; et/ou
dans lequel les ouvertures partielles (42) de l'ouverture de changement de direction (74) sont disposées dans une zone de transition (72) entre la zone d'ouverture (68) et la zone environnante (70), de telle sorte que des bords (50, 52) des ouvertures partielles (42) sont disposés dans différents plans.

13. Guidage de tuyau (36) selon la revendication 11 ou 12, dans lequel la fente (44) de l'ouverture de changement de direction (74) s'étend à travers la zone de transition (72) et la zone d'ouverture (68).

14. Composant (16, 30, 64) de système d'automatisation de laboratoire, comprenant :
un guidage de tuyau (36) selon l'une des revendications précédentes,
une pluralité de tuyaux (32) guidés à travers l'ouverture de guidage (38) ;
la plaque (40) dotée de l'ouverture de guidage (38) étant une enveloppe du composant (16, 30, 64).

15. Système d'automatisation de laboratoire (10), comprenant :
une table de travail (14) ;
un rail (26) fixé à la table de travail ;
un bras de pipetage (16) fixé au rail (26) de manière mobile par le biais d'une suspension de bras (64), sur lequel un dispositif de pipetage (28) est fixé au-dessus de la table de travail (14) ;
une pompe (30) ;
une pluralité de tuyaux (32), qui s'étendent de la pompe (30) au dispositif de pipetage (28) ;
une plaque (40) dotée d'un guidage de tuyau (36) selon l'une des revendications 1 à 13 pour les tuyaux.
